# EUROPEAN PATENT APPLICATION

(11) **EP 4 505 998 A1**
(43) Date of publication of application: **12.02.2025**
(21) Application number: 24191807.7
(22) Date of filing: 30.07.2024
(51) Int. Cl.: A61K 9/20, A61K 31/155, A61K 9/28, A61K 31/7034

(54) **PHARMACEUTICAL COMPOSITION CONTAINING DAPAGLIFLOZIN AND METFORMIN**

(30) Priority: 08.08.2023 CZ 202341213 U
(71) Applicant: Zentiva, K.S., 10200 Praha 10 - Dolni Mecholupy (CZ)
(72) Inventor: Kubelka, Tomas, Praha 9 (CZ); Pecek, Daniel, Praha 9 (CZ)
(74) Representative: Harber IP s.r.o.

(57) **Abstract**

The invention relates to a pharmaceutical formulation containing dapagliflozin or a pharmaceutically acceptable solvate thereof and metformin or a salt thereof with an inorganic or organic pharmaceutically acceptable acid, wherein said pharmaceutical formulation further contains polyvinylpyrrolidone, hydroxypropyl methyl cellulose, sodium bicarbonate and magnesium stearate as excipients.

## Description

### Field of Art

The invention concerns a pharmaceutical composition containing dapagliflozin and metformin.

### Background Art

Pharmaceutical products containing a combination of dapagliflozin and metformin are commercially available, and they are medically used to improve the condition of patients suffering from type 2 diabetes and reduce the risk of heart failure in patients suffering from type 2 diabetes in combination with heart disease or chronic liver disease.

Dapagliflozin removes excess glucose from the body through urine, and metformin reduces the production of glucose in the liver, slows the absorption of glucose in the intestines and increases the body's sensitivity to insulin.

The aim of the present invention is to provide a pharmaceutical formulation containing dapagliflozin and metformin, with the smallest possible tablet size. A small size of the tablets generally increases patient compliance with the treatment regimen because such tablets are easier to swallow.

### Disclosure of the Invention

The invention provides a pharmaceutical formulation containing dapagliflozin and metformin. The pharmaceutical formulation further contains polyvinylpyrrolidone, hydroxypropyl methyl cellulose, sodium bicarbonate (sodium hydrogencarbonate) and magnesium stearate as excipients.

Preferably, the pharmaceutical formulation contains a coating which contains hydroxypropyl methyl cellulose, talc, polyethylene glycol and optionally a dye. The dye can be, for example, yellow iron oxide or red iron oxide. The coating is mainly intended for masking the bitter taste of the active substance.

The pharmaceutical formulation is in the form of tablets or coated tablets.

The above-mentioned excipients can be used in very low amounts in the formulation of the invention, thereby allowing to reduce the total weight of each tablet, and thus to reduce the dimensions of the tablets, while maintaining good tabletability of the mixture. A particularly problematic component for tableting is metformin or salts thereof, but surprisingly, good tabletability is achieved with the composition of excipients listed here.

Dapagliflozin can be in the form of a pharmaceutically acceptable solvate, especially in the form of dapagliflozin propanediol hydrate.

Metformin may be in the form of a salt with an inorganic or organic pharmaceutically acceptable acid, particularly in the form of metformin hydrochloride.

In a preferred embodiment, the pharmaceutical formulation in the form of a tablet contains a tablet core having the following composition:
90 to 91 wt. % metformin hydrochloride,
0.5 to 0.7 wt. % dapagliflozin propanediol hydrate,
4.5 to 5 wt. % polyvinylpyrrolidone,
3 to 3.3 wt. % hydroxypropyl methyl cellulose,
0.4 to 0.5 wt. % sodium bicarbonate,
0.5 to 0.6 wt. % magnesium stearate.

The amount of metformin in the form of hydrochloride salt is preferably 850 mg or 1000 mg. The amount of dapagliflozin or a solvate thereof is preferably such that it corresponds to 5 mg of dapagliflozin.

In the case of a pharmaceutical formulation having the form of coated tablets, the core of the tablet constitutes at least 95% of the weight of the formulation, preferably 96 to 97% of the weight of the formulation.

### Examples

Procedure for preparation of pharmaceutical formulations:

All raw materials were weighed. An aqueous solution of polyvinylpyrrolidone binder was prepared. A premix of dapagliflozin propanediol hydrate with hydroxypropyl methyl cellulose and sodium bicarbonate was prepared by mixing and homogenization. Metformin hydrochloride was first deagglomerated in a high-speed mixer, then homogenized in this mixer with dapagliflozin propanediol hydrate premix, and the mixture was granulated with the aqueous solution of polyvinylpyrrolidone, freeze-dried and sieved. The lubricant magnesium stearate was mixed with the granulate. Tablet cores were pressed from the mixture. The tablet cores were coated with a coating prepared by mixing hydroxypropyl methyl cellulose, talc, polyethylene glycol, water and optionally a dye.

### Example 1: Coated tablets containing 1000 mg metformin hydrochloride and 5 mg dapagliflozin

Composition of the tablet:

| **Component** | **mg/tbl** | **wt. %, relative to tablet** | **wt. %, relative to tablet core** |
|---|---|---|---|
| Metformin hydrochloride | 1000.00 | 87.01 | 90.48 |
| Povidon 25 (polyvinylpyrrolidone) | 53.00 | 4.61 | 4.80 |
| Hypromellose K15 (hydroxypropyl methyl cellulose) | 35.20 | 3.06 | 3.19 |
| Dapagliflozin propanediol hydrate | 6.15 | 0.54 | 0.56 |
| Sodium bicarbonate | 5.00 | 0.44 | 0.45 |
| Water for granulation | q.s. | q.s. | |
| Magnesium stearate | 5.80 | 0.50 | 0.52 |
| Hypromellose E5 (hydroxypropyl methyl cellulose) | 31.40 | 2.73 | |
| Talc | 7.40 | 0.64 | |
| Macrogol 6000 (polyethylene glycol) | 5.20 | 0.45 | |
| Yellow iron oxide E172 | 0.16 | 0.01 | |
| Water for coating | q.s. | - | |
| Total weight of tablet core: | 1105.15 | 96.16 | |
| Total weight of coated tablet: | 1149.31 | 100.00 | |

The resulting tablet has the following dimensions: width: 10.1 mm; length: 20.7 mm; height: 6.4 mm. The shape of the tablet is oblong.

The weight of the coated tablet is 1149.31 mg.

A reference product Xigduo 5/1000 mg has the following dimensions: width: 10.8 mm (i.e., + 0.7 mm, compared to the product according to the invention); length: 21.8 mm (i.e., + 1.1 mm), height: 6.9 mm (i.e., + 0.5 mm). The shape of the tablet is biconvex. The weight of the coated tablet of the reference product is 1480 mg, which is almost 30% more than the tablet according to the invention.

### Example 2: Coated tablets containing 850 mg metformin hydrochloride and 5 mg dapagliflozin

Composition of the tablet:

| **Component** | **mg/tbl** | **wt. %, relative to tablet** | **wt. %, relative to tablet core** |
|---|---|---|---|
| Metformin hydrochloride | 850.00 | 87.02 | 90.49 |
| Povidon 25 (polyvinylpyrrolidone) | 45.05 | 4.61 | 4.80 |
| Hypromellose K15 (hydroxypropyl methyl cellulose) | 29.00 | 2.97 | 3.09 |
| Dapagliflozin propanediol hydrate | 6.15 | 0.63 | 0.65 |
| Sodium bicarbonate | 4.25 | 0.44 | 0.45 |
| Water for granulation | q.s. | q.s. | |
| Magnesium stearate | 4.93 | 0.50 | 0.52 |
| Hypromellose E5 (hydroxypropyl methyl cellulose) | 26.69 | 2.73 | |
| Talc | 6.29 | 0.64 | |
| Macrogol 6000 (polyethylene glycol) | 4.42 | 0.45 | |
| Water for coating | q.s. | - | |
| Total weight of tablet core: | 939.38 | 96.17 | |
| Total weight of coated tablet: | 976.78 | 100.00 | |

The resulting tablet has the following dimensions: width: 9.8 mm; length: 19.8 mm; height: 5.9 mm. The shape of the tablet is oblong.

The weight of the coated tablet is 976.78 mg.

The reference product Xigduo 5/850 mg has the following tablet dimensions: width: 9.7 mm (i.e. + 0.1 mm, compared to the product according to the invention); length: 20.3 mm (i.e. + 0.5 mm), height: 6.7 mm (i.e. + 0.8 mm). The shape of the tablet is biconvex. The weight of the coated tablet of the reference product is 1250 mg. This is again almost 30% more than for a tablet according to the invention.

## Claims

1. Pharmaceutical formulation containing dapagliflozin or a pharmaceutically acceptable solvate thereof and metformin or a salt thereof with an inorganic or organic pharmaceutically acceptable acid, wherein said pharmaceutical formulation further contains polyvinylpyrrolidone, hydroxypropyl methyl cellulose, sodium bicarbonate and magnesium stearate as excipients.

2. Pharmaceutical formulation according to claim 1, which further contains a coating containing hydroxypropyl methyl cellulose, talc, polyethylene glycol and optionally a dye.

3. Pharmaceutical formulation according to claim 1 or 2, which is in the form of tablets.

4. Pharmaceutical formulation according to claim 1 or 2, which is in the form of coated tablets, wherein the core of the tablet constitutes at least 95% of the weight of the coated tablet.

5. Pharmaceutical formulation according to any one of claims 1 to 4, wherein dapagliflozin is in the form of dapagliflozin propanediol hydrate and/or metformin is in the form of metformin hydrochloride.

6. Pharmaceutical formulation according to any one of claims 1 to 5, which contains a tablet core having the composition:
90 to 91 wt. % metformin hydrochloride,
0.5 to 0.7 wt. % dapagliflozin propanediol hydrate,
4.5 to 5 wt. % polyvinylpyrrolidone,
3 to 3.3 wt. % hydroxypropylmethylcellulose,
0.4 to 0.5 wt. % sodium bicarbonate,
0.5 to 0.6 wt. % magnesium stearate.

7. Pharmaceutical formulation according to claim 6, wherein the amount of metformin hydrochloride is 850 mg or 1000 mg and the amount of dapagliflozin propanediol hydrate is such that it corresponds to 5 mg of dapagliflozin.
